# EUROPEAN PATENT APPLICATION

(11) **EP 0 595 530 A1**
(43) Date of publication of application: **04.05.1994**
(21) Application number: 93308319.8
(22) Date of filing: 19.10.1993
(51) Int. Cl.: C08G 59/26, C08G 59/24, C07D 493/04

(54) **Epoxide resins derived from polycyclic phenols**

(30) Priority: 29.10.1992 GB 9222698
(71) Applicant: CIBA-GEIGY AG, CH-4002 Basel (CH)
(72) Inventor: Taylor, David Alan, Cambridge, CB4 3PH (GB); Ryan, Glen Richard, Cambridge, CB1 3DN (GB); Allen, Steven Andrew, Newmarket, Suffolk, CB8 8AE (GB)
(74) Representative: Sharman, Thomas

(57) **Abstract**

An epoxide resin having the formula (I): 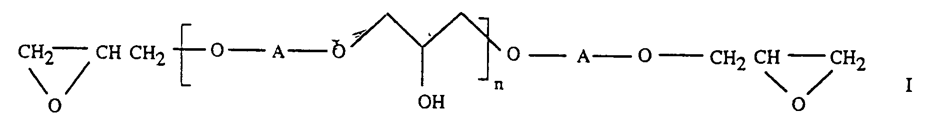
in which n is 0 or an integer ranging from 1 to 25; and A is a group having the formula II: 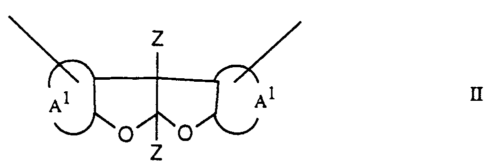
in which Z is hydrogen, methyl or phenyl; and A¹ is an organic group required to complete an aromatic residue.

## Description

The present invention relates to new epoxide resins which, on curing, have desired high thermal stability and other desirable properties.

Epoxy resins tend to absorb unduly high levels of moisture and, as a result, they are often avoided for use when exposure to heat and moisture is required.

Recently a new epoxy resin, viz. the diglycidyl ether of 9,9-bis(4-hydroxyphenyl)fluorene, has been introduced which, when cured, provides a cured material having high glass transition temperature (Tg) and flexural modulus, combined with low moisture absorption. Adhesive compositions based on this diglycidyl ether are described in U.S. Patent 4980234.

We have now found certain new epoxy resins from which cured products having still higher Tgs combined with excellent mechanical properties and low moisture absorption, can be obtained.

The present invention provides new epoxide resins having the formula (I): 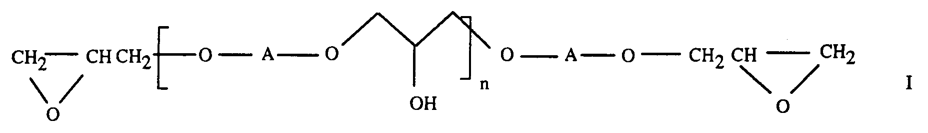
in which n is 0 or an integer ranging from 1 to 25; and A is a group having the formula II: 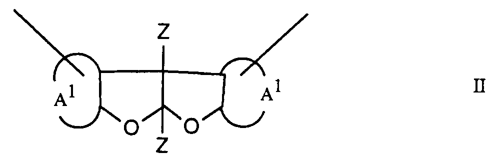
in which Z is hydrogen, methyl or phenyl; and A¹ represents an organic group required to complete an aromatic residue.

Aromatic residues completed by A¹, i.e. aromatic residues formed by A¹ together with the indicated attached carbon atoms in formula II, include phenyl, naphthyl, diphenylmethane (i.e. phenylmethylphenyl), biphenyl (i.e. biphenylyl), diphenylmethane substituted at the methane group, i.e. on the methane carbon atom, by one or two C₁-C₄ alkyl groups (i.e. phenyldi(C₁-C₄ alkyl)methylphenyl), diphenylketone (i.e. benzoylphenyl) or diphenyl sulphone (i.e. phenylsulphonylphenyl). Preferably the residue completed by A¹ is phenyl, naphthyl or diphenylmethane substituted at the methane carbon atom by two methyl groups (i.e. phenyldimethylmethylphenyl).

Examples of preferred groups of formula II include those having the formulae: 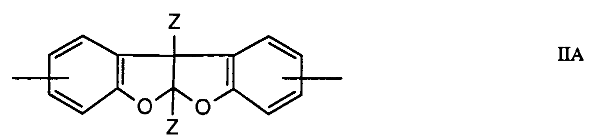
in which Z has its previous significance; 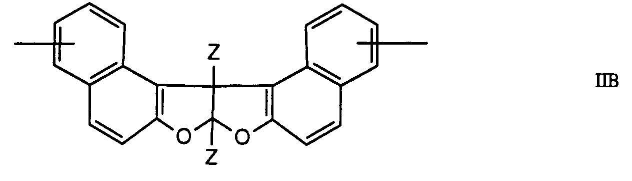
in which Z has its previous significance; 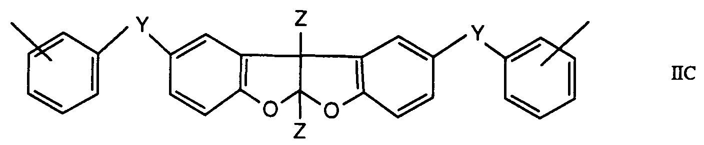
in which Z has its previous significance and Y is a direct bond; CH₂; C(C₁-C₄ alkyl)₂, preferably C(CH₃)₂; -C(=O)-; or -S(=O)₂-; and 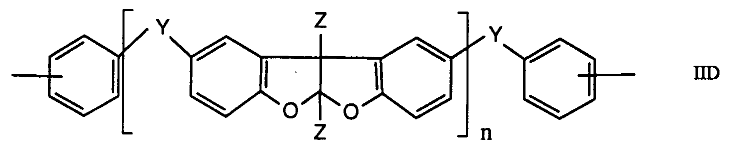
in which Z, Y and n have their previous significance.

Particularly preferred groups of formula IIA are those having the formula IIAA: 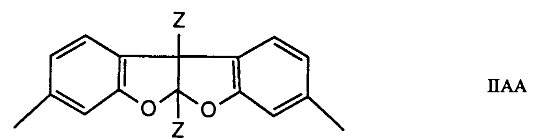
in which Z has its previous significance and is, in particular, phenyl.

Especially preferred groups of formula IIB are those having the formula IIBB: 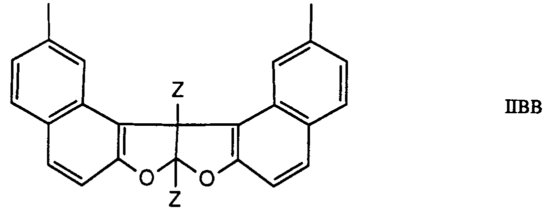
in which Z has its previous significance and preferably is hydrogen.

Particularly preferred groups of formula IIC are those of formula IICC: 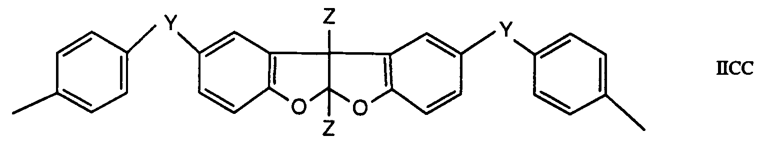
in which Y and Z have their previous significance and Z is preferably hydrogen and Y is preferably -C(CH₃)₂-.

Particularly preferred groups of formula IID are those of formula IIDD: 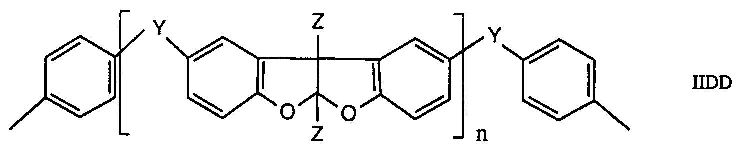
in which Z, Y and n have their previous significance and Z is preferably hydrogen and Y is preferably -C(CH₃)₃.

Preferably the value of n is so chosen that the average molecular weight of the epoxide resin of formula I ranges from 350 to 10,000.

The epoxide resins of formula I may be produced by reacting a phenol having the formula III:

HO― A― OH III

in which A has its previous signficance, with epichlorohydrin, and preferably in the presence of a catalyst; and then dehydrochlorinating the compound so obtained. The catalyst used may be a quaternary ammonium compound, a tertiary amine, a thioether or a sulphonium salt.

The proportion of epichlorohydrin used is not critical, provided that at least one equivalent per hydroxy group in the compound of formula III is used. It is convenient, however, to use epichlorohydrin as the solvent in the reaction, in which case a super-stoichiometric amount of epichlorohydrin is used, usually a total of at least 3 moles of epichlorohydrin per hydroxy group in compound of formula III.

The amount of catalyst used conveniently ranges from 0.1 to 4% by weight, based on the weight of the compound of formula III.

The reaction is normally conducted at a temperature ranging from 30 to 120°C, preferably from 40 to 80°C, and preferably under reduced pressure, to facilitate removal of water formed during the reaction.

When the phenol/epichlorhydrin reaction is judged to have been completed, the usual time required ranging from 1 to 5 hours, the reaction mixture is cooled, if necessary, to a temperature within the range of from 30° to 100°C and dehydrochlorination of the reaction product is conducted in conventional manner. Dehydrochlorination may be performed, e.g. by adding to the pheno/epichlorohydrin addition product, an alkali metal hydroxide, in particular sodium hydroxide or potassium hydroxide, optionally together with a quaternary ammonium halide, e.g. tetramethylammonium chloride or benzyltrimethylammonium chloride, as catalyst. If desired, the dehydrochlorination reaction may be performed in the presence of a solvent e.g. 2-methoxyethanol, isodecanol, ethylene glycol, diethylene glycol, N-methylpyrrolidone, gamma-butyrolactone, benzyl alcohol, dibutyl phthalate, methyl ethylketone or toluene. The dehydrochlorinating agent is preferably added portionwise, preferably in solid form, over an extended period, e.g. over a period ranging from 10 minutes to 6 hours.

The dehydrochlorination reaction mixture may be worked up in conventional manner e.g. by washing with water and separating and purifying, e.g. by distillation, the organic phase containing the desired glycidyl ether product.

The compounds of formula III, used to produce the epoxide resins of formula I, are known compounds.

Thus, compounds having the formula III in which A is a group of formula IIA in which Z is hydrogen methyl or phenyl and n is 0, have been described in Receuil 87, 1968, pages 599 to 608; compounds of formula III in which A is a group of formula IIA, in which Z is phenyl and n is 0, have been disclosed in German Offenlegungsschrift No. 3938282; and compounds of formula III in which A is a group of formula IIIB, in which Z is hydrogen and n is 0, or A is a group IIIC and Y is C(CH₃)₂ and n is 1,2 or 3, are described by Maravigna, Journal of Polymer Science; Part A: Polymer Chemistry, 26, 2475-2485.

The compounds of formula III may be produced by the methods described in the above-mentioned Receuil and Journal of Polymer Science references, and by the process disclosed in German Offenlegungsschrift No. 3938282.

In principle, compounds of formula III are produced by reacting an appropriate diol and an appropriate diketone in the presence of a strong acid, such as sulphuric acid or methane sulphonic acid, thereby providing a corresponding compound of formula III.

The present invention also provides a curable composition comprising an epoxide resin of formula I and a curing agent for the resin of formula I.

As examples of curing agents may be mentioned aliphatic, cycloaliphatic, aromatic, and heterocyclic amines such as m- and p-phenylenediamine, bis(4-aminophenyl)methane, aniline-formaldehyde resins, bis(4-aminophenyl)sulphone, ethylenediamine, propane-1,2-diamine, propane-1,3-diamine, N,N-diethylethylenediamine, hexamethylenediamine, diethylenetriamine, triethylenetetramine, tetraethylenepentamine, N-(2-hydroxyethyl)-,N-(2-hydroxypropyl)-, and N-(2-cyanoethyl)-diethylenetriamine, 2,2,4-trimethylhexane-1,6-diamine, 2,3,3-trimethylhexane-1,6-diamine, m-xylylenediamine, N,N-dimethyl- and N, N-diethylpropane-1,3-diamine, ethanolamine, bis(4-aminocyclohexyl)methane, 2,2-bis(4-aminocyclohexyl)propane, 2,2-bis(4-amino-3-methylcyclohexyl)propane, 3-aminomethyl-3,5,5-trimethylcyclohexylamine(isophoronediamine), and N-(2-aminoethyl)piperazine; dicyandiamide; carboxylic acid hydrazides; imidazoles; aminoplasts; polyaminoamides, e.g. those prepared from aliphatic polyamines and dimerised or trimerised unsaturated fatty acids; adducts of amines with stoichiometric deficits of polyepoxides such as a diglycidyl ether; isocyanates and isothiocyanates; polyhydric phenols, e.g. resorcinol, hydroquinone, 2,2-bis(4-hydroxyphenyl)propane, phenol-aldehyde resins, and oil-modified phenol-aldehyde resins; phosphoric acid; polythiols such as the "Thiokols" ("Thiokol" is registered trade mark); and polycarboxylic acids and their anhydrides, e.g. phthalic anhydride, tetrahydrophthalic anhydride, methylendomethylenetetrahydrophthalic anhydride, nonenylsuccinic anhydride, dodecenylsuccinic anhydride, hexahydrophthalic anhydride, hexachloroendomethylenetetrahydrophthalic anhydride and endomethylenetetrahydrophthalic anhydride and their mixtures, maleic anhydride, succinic anhydride, pyromellitic acid dianhydride, benzophenone-3,3', 4,4'-tetracarboxylic acid dianhydride, polysebacic anhydride, polyazelaic anhydride, the acids corresponding to the aforementioned anhydrides, and also isophthalic acid, terephthalic acid, citric acid, and mellitic acid. Particularly preferred polycarboxylic acid or anhydride curing agents are those which, in admixture if necessary, are liquid at temperatures below 60°C. There may also be used catalytic polymerising agents, such as tertiary amines (for example 2,4,6-tris(dimethylaminomethyl)phenol and other Mannich bases, N-benzyldimethylamine, and triethanolamine); alkali metal alkoxides of alcohols (for example, the sodium alcoholate of 2,4-dihydroxy-3-hydroxymethylpentane), stannous salts of alkanoic acids (for example, stannous octanoate), Friedel-Crafts catalysts such as boron trifluoride and its complexes; and chelates formed by reaction of boron trifluoride with, e.g. 1,3-diketones.

In conjunction with the curing agents there may also be used appropriate accelerators. When poly(aminoamides), dicyandiamides, polythiols, or polycarboxylic acid anhydrides are employed for curing, tertiary amines or their salts, quaternary ammonium compounds, or alkali metal alkoxides can serve as accelerators. Examples of specific accelerators are N-benzyldimethylamine, 2,4,6-tris(dimethylaminomethyl)phenol, imidazoles, and triamylammonium phenoxide.

Other accelerators which may be used include metal nitrates, particularly magnesium nitrate and manganese nitrate, fluorinated and chlorinated carboxylic acids and their salts, such as magnesium trifluoroacetate, sodium trifluoroacetate, magnesium trichloroacetate, and sodium trichloroacetate, trifluoromethanesulphonic acid and its salts, such as the manganese, zinc, magnesium, nickel and cobalt salts, and magnesium perchlorate and calcium perchlorate.

An effective amount of the curing agent is employed. The proportion will depend on the chemical nature of the curing agent and the properties sought of the curable composition and its cured product; the optimum proportion can readily be determined by methods familiar to those skilled in the art. By way of illustration, however, when the curing agent is an amine there will normally be used from about 0.75 to 1.25 amino-hydrogen equivalents of the amine per 1,2-epoxy equivalent of the epoxide resin. When polycarboxylic acids or their anhydrides are used, usually from about 0.4 to 1.1 carboxylic acid, or carboxylic acid anhydride, equivalents are taken per 1,2-epoxy equivalent, while, with polyhydric phenols about 0.75 to 1.25 phenolic hydroxy equivalents of the curing agent per 1,2-epoxy equivalent are employed. Generally, from 1 to 40 parts by weight of a catalytic polymerising agent are used per 100 parts by weight of the epoxide resin.

Curing can be carried out, depending on the nature of the curing agent, at room temperature (for example 18°C to 25°C) or at higher temperature (50° to 250°C, for example).

If desired, curing or hardening can be carried out in two stages, for example by interrupting the curing reaction or, if a curing agent requiring an elevated temperature for complete curing is used, by only partially curing at a lower temperature, to give a still fusible and soluble, curable precondensate or "B-stage" product, for use in making moulding powders, sinter-coating powders, or prepregs in a manner known per se.

The compounds of this invention may be used with conventional epoxide resins.

In the usual methods of manufacturing many epoxide resins, mixtures of compounds of differing molecular weight are obtained, these mixtures ordinarily containing a proportion of compounds whose epoxide groups have undergone partial hydrolysis or in which epoxidation has not proceeded to completion. The average number of 1,2-epoxide groups per molecule of an epoxide resin need not be at least 2 and need not be integral; it is generally a fractional number, but must in any case be greater than 1.0.

Of the epoxide resins which may be used in admixture with the glycidyl ethers of formula I the more suitable are those wherein the epoxide groups are also terminal, i.e. of formula 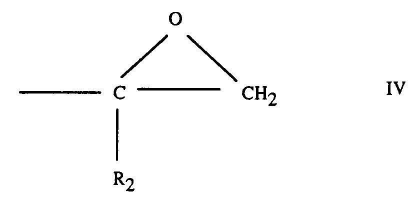
where R² denotes a hydrogen atom or a methyl group, and especially those where the groups are present as glycidyl or β-methylglycidyl groups directly attached to an atom of oxygen, nitrogen, or sulphur. Accordingly, the invention also provides a resin of formula I as hereinbefore defined in admixture with a further epoxide resin having, on average, more than one group of formula IV per molecule. Resins containing a group of formula IV include polyglycidyl and poly(β-methylglycidyl) esters obtainable by the reaction of a substance containing two or more carboxylic acid groups per molecule with epichlorohydrin, glycerol dichlorohydrin, or β-methylepichlorohydrin in the presence of alkali. The polyglycidyl esters may be derived from aliphatic carboxylic acids, e.g. oxalic acid, succinic acid, adipic acid, sebacic acid, or dimerised or trimerised linoleic acid, from cycloaliphatic carboxylic acids such as hexahydrophthalic, 4-methylhexahydrophthalic, tetrahydrophthalic, and 4-methyltetrahydrophthalic acid, or from aromatic carboxylic acids such as phthalic acid, isophthalic acid, and terephthalic acid.

Other epoxide resins which may be used include polyglycidyl and poly(β-methylglycidyl) ethers obtainable by the reaction of substances containing per molecule, two or more alcoholic hydroxy groups, or two or more phenolic hydroxy groups, with epichlorohydrin, glycerol dichlorohydrin, or β-methylepichlorohydrin, under alkaline conditions or, alternatively, in the presence of an acidic catalyst with subsequent treatment with alkali. Such polyglycidyl ethers may be derived from aliphatic alcohols, for example, ethylene glycol and poly(oxyethylene) glycols such as diethylene glycol and triethylene glycol, propylene glycol and poly(oxypropylene) glycols, propane-1, 3-diol, butane-1, 4-diol, pentane-1, 5-diol, hexane-1, 6-diol, hexane-2,4,6-triol, glycerol, 1,1,1-trimethylolpropane, and pentaerythritol; from cycloaliphatic alcohols, such as quinitol, 1,1-bis(hydroxymethyl)cyclohex-3-ene, bis(4-hydroxycyclohexyl)methane, and 2,2-bis(4-hydroxycyclohexyl)propane, or from alcohols containing aromatic nuclei, such as N,N-bis(2-hydroxyethyl)aniline and 4,4'-bis(2-hydroxyethylamino)diphenylmethane. Preferably the polyglycidyl ethers are derived from substances containing two or more phenolic hydroxy groups per molecule, for example, resorcinol, catechol, hydroquinone, bis(4-hydroxyphenyl)methane, 1,1,2,2-tetrakis(4-hydroxyphenyl)ethane, 4,4'-dihydroxydiphenyl, bis(4-hydroxyphenyl)sulphone, and especially, phenol-formaldehyde or cresol-formaldehyde novolac resins, 2,2-bis(4-hydroxyphenyl)propane (otherwise known as bisphenol A), and 2,2-bis(3,5-dibromo-4-hydroxyphenyl)-propane.

There may further be employed poly(N-glycidyl)compounds, such as are, for example, obtained by the dehydrochlorination of the reaction products of epichlorohydrin and amines containing at least two hydrogen atoms directly attached to nitrogen, such as aniline, n-butylamine, bis(4-aminophenyl)methane, bis(4-aminophenyl)sulphone, and bis(4-methylaminophenyl)methane. Other poly(N-glycidyl)compounds that may be used include triglycidyl isocyanurate, N,N'-diglycidyl derivatives of cyclic alkylene ureas such as ethyleneurea and 1,3-propyleneurea, and N,N'-diglycidyl dervivatives of hydantoins such as 5,5-dimethyl-hydantoin.

Epoxide resins obtained by the epoxidation of cyclic and acyclic polyolefins may also be employed, such as vinylcyclohexene dioxide, limonene dioxide, dicylopentadiene dioxide, 3,4-epoxydihydrodicylopentadienyl glycidyl ether, the bis(3,4-epoxydihydrodicylopentadienyl) ether of ethylene glycol, 3,4-epoxycyclohexylmethyl-3', 4'-epoxycyclohexanecarboxylate and its 6,6'-dimethyl, derivative, the bis(3,4-epoxycyclohexanecarboxylate) of ethylene glycol, the acetal formed between 3,4-epoxycyclohexanecarboxyladehyde and 1,1-bis(hydroxymethyl)-3, 4-epoxycyclohexane, bis(2,3-epxoycyclopentyl)ether, and epoxidized butadiene or copolymers of butadiene with ethylenic compounds such as styrene and vinyl acetate.

Especially suitable epoxide resins for mixing with the glycidyl ethers of formula I are polglycidyl ethers of 2,2-bis(4-hydroxyphenyl)propane or a novolac from phenol (which may be substituted in the ring by a chlorine atom or an alkyl group of from 1 to 4 carbon atoms) and formaldehyde and having an epoxide content of at least 1.0 1,2-epoxide equivalent per kilogram.

The compositions of the invention may further contain plasticisers such as dibutyl phthalate, dioctyl phthalate, or tricresyl phosphate, inert diluents, and so-called reactive diluents, such as diglycidyl formal and especially monoepoxides such as butyl glycidyl ether, iso-octyl glycidyl ether, phenyl glycidyl ether, styrene oxide, glycidyl acrylate, glycidyl methacrylate, and glycidyl esters of synthetic, highly branched, predominantly tertiary, aliphatic monocarboxylic acids. They may also contain additives such as fillers, reinforcing materials, colouring matter, flow control agents, flame retardants, and mould lubricants. Suitable extenders, fillers, and reinforcing materials include asbestos, asphalt, bitumen, glass fibres, textile fibres, carbon fibres, boron fibres, mica, alumina, gypsum, titania, chalk, quartz flour, cellulose, kaolin, ground dolomite, wollastonite, collodial silica having a large specific surface such as that available under the registered trademark "Aerosil", clays modified by treatment with long chain amines (such as those available under the registered trade mark "Bentone"), powdered poly(vinyl chloride), powdered polyolefin hydrocarbons, powdered cured aminoplasts, and metal powders such as aluminium or iron powder. Flame retardants such as antimony trioxide may also be incorporated.

The curable compositions of this invention may be used as adhesives, primers for adhesives, laminating resins, impregnating and casting resins, moulding compositions, putties and sealing compounds and potting and insulating compounds for the electrical industry.

The compositions of this invention may be cured by heating them at a suitable temperature, viz. 0 to 250°C, which will vary depending on the nature of the curing agent. The length of the curing process will also vary according to the nature of the curing agent but may range from 5 minutes to 7 days.

The present invention also provides cured compositions obtained by curing the curable compositions of the present invention.

The following Examples further illustrate the present invention.

### Example 1

A) In the manner defined in J. Polymer Science; Part A: Polymer Chem. 1985, 26, 2475 0.01 mol of 2,7-dihydroxynaphthalene, dissolved in 10ml of acetic acid and 0.01 mol of glyoxal (aqueous solution), is mixed into a reaction vessel under nitrogen, at 25-30°C. 2ml of 96% H₂SO₄ are added, dropwise, with stirring. The compound having the formula: 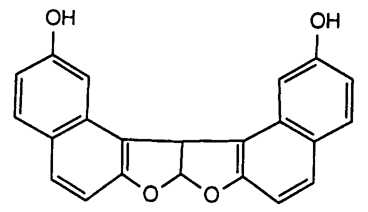 is obtained in quantitative yield and crystallises from ethanol to give a m. pt of 304°C (dec).
B) A solution of the bisphenol (1.0 mol) from Part A and 50% aqueous tetramethylammonium chloride (3 wt % of the bis-phenol), in epichlorohydrin (10 mol), is refluxed, under nitrogen, for 3 hours. The solution is cooled to 60°C and sodium hydroxide (2.25 mol) is added, in portions, over 2 hours. The mixture is stirred at 75°C for 1 hour. Water is added to dissolve the precipitated salt, and the mixture is washed with sodium dihydrogen phosphate (10%). The organic phase is separated and dried. Removal of the solvent under reduced pressure affords the diglycidyl ether having the formula: 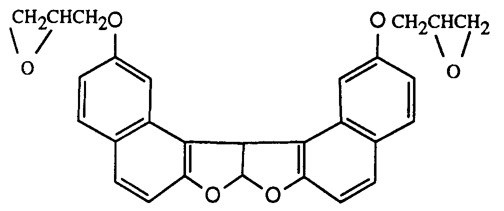 (yield 80% of the theoretical), m.p. (DSC) 160°C, epoxide content 3.81 mol kg⁻¹.
   IR 1660, 1600, 1515, 1465, 1450, 1375, 1312, 1255, 1210, 1130, 1065, 960, 910, 838 cm⁻¹.
   ¹H-NMR (DMSO, 250 MHz) 2.72, m, 1H; 2.78, m, 1H; 2.89, m, 2H; 3.34, m, 2H; 3.62, m, 1H; 3.91, m, 1H; 4.22, m, 1H; 4.46, m, 1H; 5.80, d, J 5.7 Hz, 1H; 7.08, d, J9.0 Hz, 2H; 7.14, d, J8.6 Hz, 2H; 7.28, d, J5.7 Hz, 1H; 7.68, m, 2H; 7.79, d, J8.6 Hz, 2H; 7.85, d, J9.0 Hz, 2H.

### Example 2

A) In the manner described in German Offenlegungsschrift No. 3938282, a mixture of 743g resorcinol, 756 g of benzil and 2.0 kg of xylene is saturated with HCl gas at 50-60°C and stirred for 5-6 hours at this temperature. The product so obtained is washed several times with xylene and vacuum - dried at 100°C to give 1103 g (81% theoretical yield) of a compound having the formula: 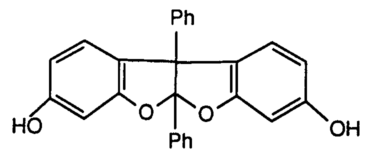 having m. pt. 260-262°C.
B) The bisphenol obtained in Part A) is then glycidylated, as described in Example 1(B), to give a diglycidyl ether having the formula: 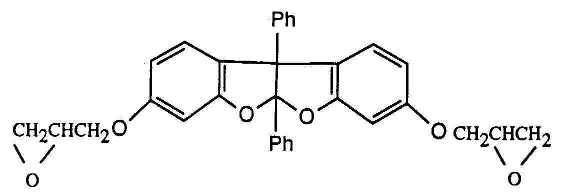 in a yield of 80% of the theoretical, m.p. 120°C, epoxide content 3.18 mol kg⁻¹.
   The crude product is purified by triturating with diethyl ether to afford a white powder, m.p. 180°C, epoxide content 3.48 mol kg⁻¹.
   ¹H-NMR (acetone, 250MHz) 2.73, m, 2H; 2.85, m, 2H; 3.34, m, 2H; 3.92, dd, J 11.3 Hz and 6.4 Hz, 2H; 4.38, m, 2H; 6.68, dd, J 8.3 Hz and 2.3 Hz, 2H; 6.77, m, 4H; 7.00-7.20, m, 10H.
   IR 1625, 1605, 1500, 1450, 1330, 1285, 1170, 1035, 985, 760, 700cm⁻¹.

### Example 3

The product of Example 1(B) is cured with 4,4'-diaminodiphenylsulphone (1:1 stoichiometry). The gel time is 17 minutes at 180°C and 11 minutes at 210°C. Tₒₙₛₑₜ (the temperature at which onset of gelation begins) is about 170°C. Glass transition temperature (Tg) results, as measured by thermomechanical analysis (TMA), and decomposition onset temperature (Td) results as measured by thermogravimetric analysis (TGA) for different cure cycles are as follows:

| Cure Cycle | Tg (TMA) | Td (TGA) |
|---|---|---|
| 2h/180°C + 2h/210°C | 229°C | 385°C |
| 2h/180°C + 4h/210°C | 231°C | 380°C |
| 2h/210°C | 227°C | 375°C |
| 4h/210°C | 232°C | 360°C |

When the product of Example 1(B) is cured with alkylated 4,4'-diaminodiphenylmethane (1:1 stoichiometry) the gel time is 3 minutes at 180°C.

| Cure Cycle | Tg (TMA) | Td (TGA) |
|---|---|---|
| 4h/180°C | 200°C | 330°C |
| 4h/180°C + 2h/210°C | 200°C | 330°C |

### Example 4

The crude product of Example 2(B) is cured with 4,4¹-diaminodiphenylsulphone (1:1 stoichiometry). The gel time at 180°C is 15 minutes and Tₒₙₛₑₜ is 185°C.

| Cure Cycle | Tg (TMA | Td (TGA) |
|---|---|---|
| 4h/180°C | 169°C | 265°C |
| 4h/180°C + 2h/210°C | 175°C | |

Purified material cured with diaminodiphenylsulphone (1:1 stoichiometry) for 3 hours at 210°C gives a Tg of 213°C.

### Example 5

A) In the manner described in Example 1(A), bisphenol A is reacted with glyoxal, using H₂SO₄ as catalyst, to produce the compound of formula: 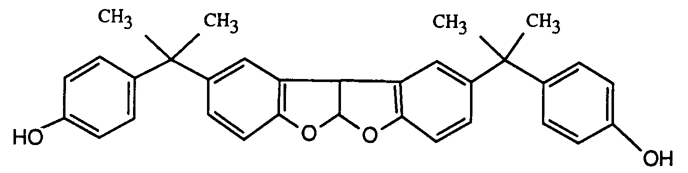 having m. pt. 140-170°C.
   ¹H-NMR (acetone - d₆ 250 MHz) 1.57, m, 12H; 5.00, m, 1H; 6.65-6.80, m, 4H; 6.91, d, J 6.7 Hz, 1H; 6.91-7.30, m, 10H; 8.19, s, 2H.
   ¹³C-NMR (62.9 MHz, acetone-d₆) 31.72, 31.93, 42.73, 51.17, 109.65, 114.28, 115.79, 124.01, 127.60, 128.71, 128.84, 142.60, 146.34, 156.32, 156.78.
   IR 3400, 2970, 1615, 1595, 1515, 1495, 1245, 1180, 980, 830, cm⁻¹.
   Varying the amount of glyoxal used in Example 5(A) does not change the nature of the product obtained in Example 5(A). Thus, use of phenol: glyoxal ratios of 2:1, 1:1 or 3:2, respectively, always produces the dimeric product shown in Example 5(A).
B) The product of Part (A) is glycidylated using the procedure described in Example 1(B) to give a 95% theory yield of a diglycidyl ether having the formula: 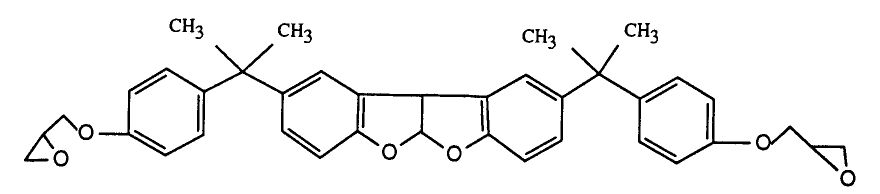
   Epoxide content: 3.6 mol kg⁻¹, as a dark gum.
   ¹H-NMR (250 MHz, acetone d₆) 1.55, m, 12H; 2.67, m, 2H; 2.82, m, 2H; 3.29, m, 2H; 3.80, m, 2H; 4.26, m, 2H; 5.00, m, 1H; 6.65-7.40, m, 15H.
   ¹³C-NMR (62.9 MHz, acetone - d₆) 31.03, 31.93, 42.86, 44.68, 50.90, 51.16, 70.27, 109.76, 114.30, 115.03, 124.03, 127.64, 128.80, 128.90, 144.33, 146.05, 156.85, 157.80.
   IR 2965, 1610, 1580, 1515, 1497, 1245, 1030, 975, 830 cm⁻¹.

### Example 6

When the product of Example 5(B) is cured with diaminodiphenylsulphone (1:1 stoichiometry) the following results are obtained: gel time at 180°C: 12 minutes; Tₒₙₛₑₜ (DSC) : 170°C.

| Cure Cycle | Tg (TMA) | Td (TGA) |
|---|---|---|
| 4h at 180°C | 180°C | 360°C |
| 4h at 180°C + 2h^{@} 210°C | 190°C | 360°C |
| 2.5h at 210°C | 220°C | 360°C |

### Example 7

A) In the manner described in Example 1(A), bisphenol-A (20g, 0.087 mol) and 35% aqueous glyoxal (9.6g, 0.057 mol) are reacted using methane sulfonic acid (63ml) as the catalyst. The reaction mixture is stirred at room temperature for 18 hours and then poured into water. The resulting precipitate is worked up to afford a polymeric solid (Mn 1125, Mw/Mn 2.8) of formula 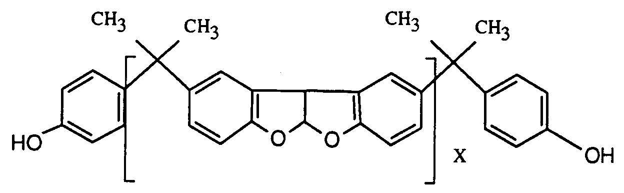
   An increased amount of glyoxal relative to bisphenol A results in products of higher molecular weight.
B) The product of Part (A) is glycidylated using the procedure described in Example 1 (B) to give a diglycidyl ether having the formula: 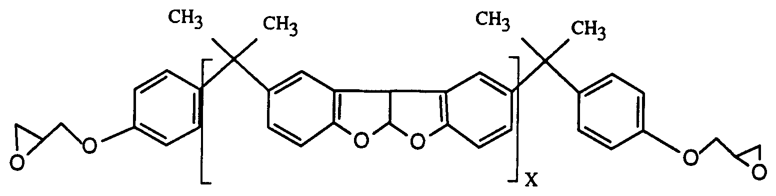
   Epoxide content: 2.30 mol kg⁻¹.

## Claims

1. An epoxide resin having the formula (I): 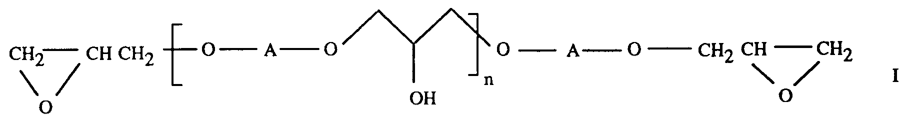 in which n is 0 or an integer ranging from 1 to 25; and A is a group having the formula II: 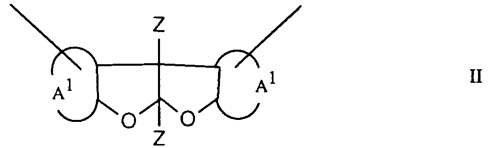 in which Z is hydrogen, methyl or phenyl; and A¹ is an organic group which completes an aromatic residue.

2. A resin according to claim 1, in which the residue completed by A¹ is phenyl, naphthyl, biphenylyl, phenylmethylphenyl, phenyl-di(C₁-C₄ alkyl)methylphenyl, benzoylphenyl or phenylsulfonylphenyl.

3. A resin according to claim 2, in which said residue is phenyl, naphthyl or phenyl(dimethyl)methylphenyl.

4. A resin according to claim 1, in which A has the formula: 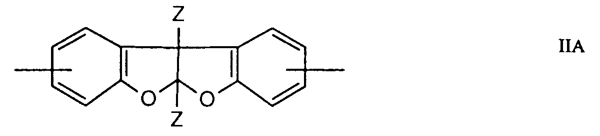 or 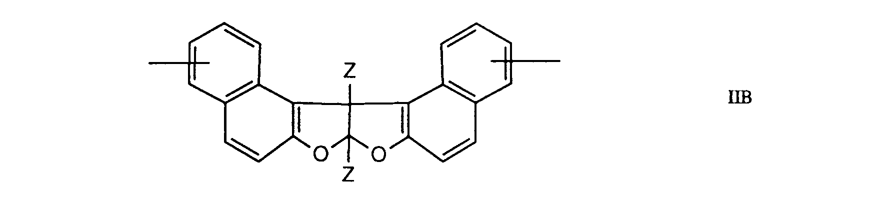 or 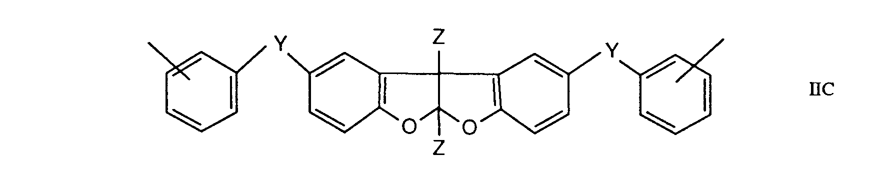 or 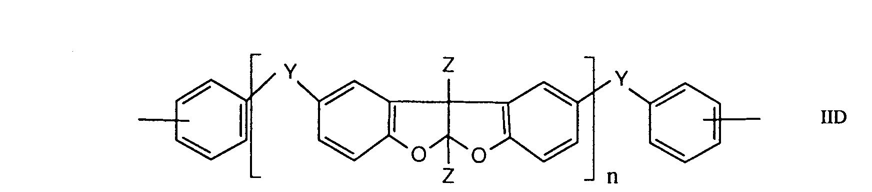 in which Z and n are as defined in claim 1 and Y is a direct bond, CH₂, C(C₁-C₄ alkyl)₂, C(=O) or -S(=O)₂.

5. A resin according to claim 4, in which A has the formula: 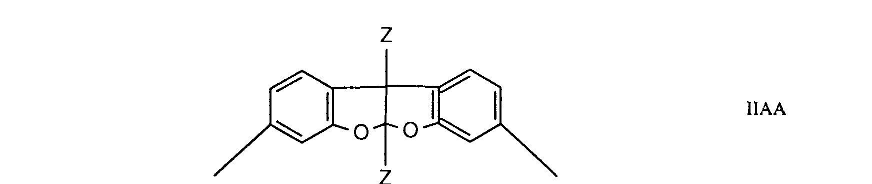 or 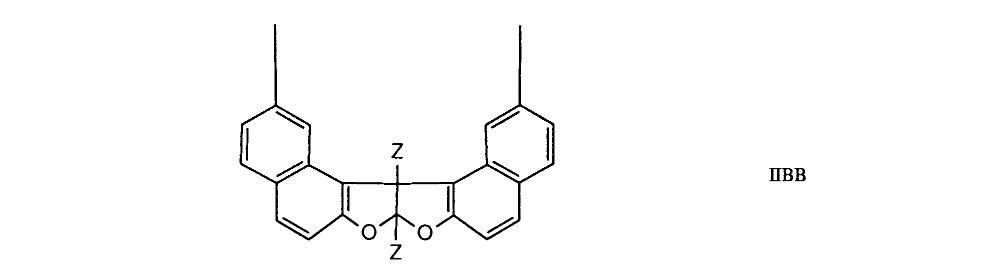 or 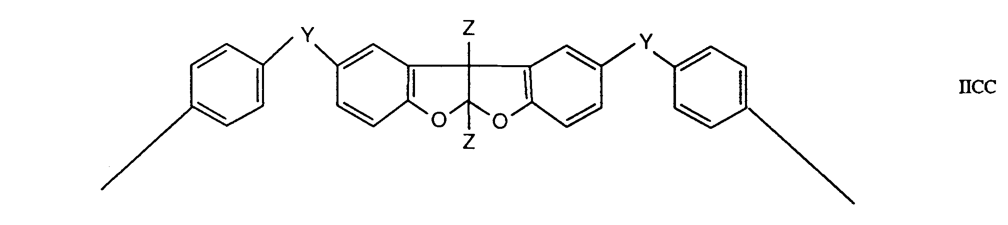 or 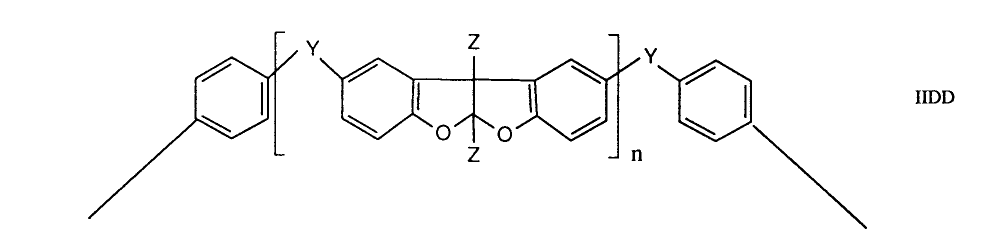 in which Y, Z and n are as defined in claim 4.

6. A resin according to claim 5, in which A is of formula IIAA where Z is phenyl, or A is of formula IIBB where Z is hydrogen, or A is of formula IICC where Z is hydrogen and Y is -C(CH₃)₂, or A is of formula IIDD where Z is hydrogen and Y is -C(CH₃)₂.

7. A resin according to any of claims 1 to 6 in admixture with a further epoxide resin having, on average, more than one group of formula IV 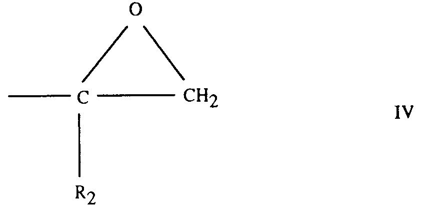 per molecule, where R² is a hydrogen atom or a methyl group.

8. A curable composition comprising 1) an epoxide resin of formula I, according to any of claims 1 to 6; and 2) a curing agent for the epoxide resin of formula I.

9. A composition according to claim 8, which also contains a further epoxide resin having, on average, more than one group of formula IV 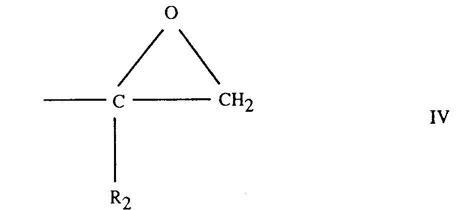 per molecule, where R² is a hydrogen atom or a methyl group.

10. A cured product obtained by curing a curable composition according to claim 8 or 9.
